# EUROPEAN PATENT SPECIFICATION

(11) **EP 3 078 702 B1**
(45) Date of publication and mention of the grant of the patent: **16.09.2020**
(21) Application number: 16163856.4
(22) Date of filing: 05.04.2016
(51) Int. Cl.: C08J 5/18, C08J 7/00, C08J 7/04, A61B 6/04

(54) **SILICONE COVERED SHEET FOR RADIOTHERAPY APPLICATIONS**
SILIKONBESCHICHTETE FOLIE FÜR STRAHLENTHERAPIEANWENDUNGEN
FEUILLE RECOUVERTE DE SILICONE POUR DES APPLICATIONS DE RADIOTHERAPIE

(30) Priority: 08.04.2015 EP 15162734; 30.04.2015 EP 15166056; 17.06.2015 EP 15172528
(43) Date of publication of application: 12.10.2016
(73) Proprietor: T Tape Company BV, 4645 EX Putte (NL)
(72) Inventor: Anthony, Jean-Michel, 2950 Kapellen (BE); Nieberding, Reginald, 2950 Kapellen (BE)
(74) Representative: Brantsandpatents bvba

(56) References cited:
- WO-A1-2013/167688
- WO-A2-2014/170220

## Description

### TECHNICAL FIELD

The present invention pertains to a thermoplastic sheet for immobilizing at least part of a human's body part for receiving radiation treatment. The invention further pertains to a method for the production of said sheet. The invention is suitable for use in the medical field, particularly for immobilization purposes in radiotherapy cancer treatment.

### BACKGROUND

The treatment of patients having cancer frequently makes use of radiation therapy wherein radiation is directed to particular sites in the patient's body. These treatments require high precision, reliable and accurate patient set-up to position and immobilize the relevant portion of the patient's body undergoing the radiation. Patients being treated by radiotherapy are typically required to receive radiation doses at regular intervals, whereby each dose should be precisely directed to the same location of the body. This necessitates immobilization materials and devices that are adapted to the shape of the body portion being treated and that allow reproducible immobilization of said body part.

Thermoplastic sheets are well-known in the medical field, particularly for their use as shaped articles for immobilizing a body part. A commonly used body part, i.e. the head, restraint device is a mask that is placed over the face of the patient to hold the patient's head stationary. Such masks may be obtained by molding a thermoplastic sheet on a patient's face to ensure maximum immobilization. The back of the patient's head and/or contiguous portion of the patient's neck may be supported by a cushion. The molding of the sheet is typically conducted preceding the first treatment. Afterwards and for subsequent treatments, the mask can repeatedly be mounted on the head of the patient and fixed to a coating table. Such masks are disclosed for example in WO 2014/170220 A2 or WO 2013/167688 A1.

One of the disadvantages of the thermoplastic sheets of the prior art is their thickness. A minimum rigidity of the sheet is required so that the sheet maintains the molded shape and does not retract after molding. Said rigidity is conferred by the thickness of the thermoplastic sheet: the higher the thickness is the better is the rigidity of the molded sheet. However, the higher the thickness is, the lower is the quality of the molding in terms of conformance to the anatomical contours of the body part. In most cases a relatively high pressure is applied on the heated sheet by the practitioner so to conform the heated sheet to the anatomical contours of the body part to be immobilized. Even when applying such pressure during the molding step, the sheet will not perfectly conform the anatomical contours of the body part especially in irregular areas such as the nose, the chin and the eyes contours. Therefore, a remaining challenge is to provide a thermoplastic sheet which is as thin as possible to easily and without applying a high pressure conform the anatomical contours of a body part while having the required rigidity for maintaining the molded form with minimal or no retraction after molding and curing.

Another disadvantage of the thermoplastic sheets of the prior art is their effect on the dosimetry of the applied radiotherapy. Indeed, thick sheets and/or sheets made of certain material will have influence on the radiation dosimetry and/or cause a higher attenuation and/or an increased radiation dose on the patient's skin and/or radiation scattering. This considerably reduces the effectiveness of the treatment delivered to the patient.

The aim of the present invention is to provide a solution to overcome at least part of the above mentioned disadvantages. The main objective is to provide a thermoplastic sheet presenting high performance in terms of radiation therapy and offering maximal comfort to the practitioner and the patient. Other objectives will become apparent from the description of the invention. The thermoplastic sheet of the invention solves the above mentioned problems and is as described in the description and in the appended claims.

### SUMMARY OF THE INVENTION

In a first aspect, the present invention provides a thermoplastic sheet for use in radiotherapy treatment, comprising a thermoplastic composition layer comprising a mixture of polycaprolactone and polyurethane, said layer is provided with an upper surface and a lower surface; and a support layer provided on at least one surface of the thermoplastic composition layer, said support layer comprises silicone which is at least partially polymerized, characterized in that, the thickness of the thermoplastic composition layer is at most 1.8 mm, the thickness of the support layer is of from 80 µm to 180 µm, and the radiation transmission factor of the sheet is at least 80%.

In a second aspect, the present invention provides a method for the production of a thermoplastic sheet. Said sheet is as described above. The method comprises the steps of:
- producing a thermoplastic composition layer comprising a mixture of polycaprolacone and polyurethane,
- treating at least one surface of the thermoplastic composition layer thereby increasing its surface adherence,
- applying a support layer on the treated thermoplastic composition surface wherein said support layer comprises silicone, and
- creating spaced apart groups of perforations.

Using the production method of the present invention, the support layer of the sheet does not peel off upon heating the sheet for molding, after a first use or after repetitive use.

Also provided is the use of the thermoplastic sheet according to any embodiment of the present invention for molding a mask directly on a person body part, whereby the support layer is the skin-facing layer.

The invention further provides a kit comprising at least one thermoplastic sheet according to any embodiment of the present invention and at least one leaflet comprising use instructions.

The invention presents several advantages. The thermoplastic sheet is thin thereby providing good molding properties and excellent conformance to the anatomical contours of the body part to be immobilized. At the same time, the thin sheet of the invention has sufficient rigidity to maintain the molded shape and to reduce and avoid retraction after the molding step. The rigidity of the sheet of the present invention is comparable and similar to the rigidity of a thermoplastic sheet having a thickness of 2.3 mm while the sheet of the present invention is thinner, i.e. at most 1.8 mm. Said thickness is the thickness of the sheets prior application on the patient's body part to be immobilized. Another advantage of the sheet of the present invention is that retraction of the sheet after molding is considerably reduced and even inexistent thereby avoiding any pressure of the molded sheet on the immobilized body part.

The sheet of the invention has high radiation transmission factor which does not influence and improves the radiation dosimetry. This is of outmost importance for the effectiveness of the repetitive radiotherapy treatment.

The support layer of the sheet of the invention improves the conformance of the sheet to the anatomical contours of the body part thereby accelerating the molding process and shortening the molding time. Indeed the intervention of the practitioner during molding is considerably reduced as applying a pressure on the heated sheet is not required for reaching good molding properties. The sheet is also transparent thereby allowing the practitioner to visually follow the molding quality of the sheet on the patient's body part. The practitioner's work and the patient's contort are thereby increased. The sheet of the invention has also a longer life time compared to the sheets of the prior art.

### DESCRIPTION OF FIGURES

**Figure 1** shows a top view of the non molded sheet according to any embodiment of the present invention.
**Figure 2** shows a top view of the molded sheet according to any embodiment of the present invention.
**Figure 3** shows a top view of the molded sheet with fixation elements according to any embodiment of the present invention.
**Figure 4** shows a side view of the molded sheet with fixation elements according to any embodiment of the present invention.
**Figure 5** shows an embodiment of the invention wherein the sheet of Figure 4 is fixed to a fixation surface and wherein a cushion is used for immobilizing a patient's head.

### DETAILED DESCRIPTION OF THE INVENTION

Unless otherwise defined, all terms used in disclosing the invention, including technical and scientific terms, have the meaning as commonly understood by one of ordinary skill in the art to which this invention belongs. By means of further guidance, term definitions are included to better appreciate the teaching of the present invention.

"A", "an", and "the" as used herein refers to both singular and plural referents unless the context clearly dictates otherwise. By way of example, "a compartment" refers to one or more than one compartment.

"About" as used herein referring to a measurable value such as a parameter, an amount, a temporal duration, and the like, is meant to encompass variations of +/-20% or less, preferably +/-10% or less, more preferably +/-5% or less, even more preferably +/-1% or less, and still more preferably +/-0.1% or less of and from the specified value, in so far such variations are appropriate to perform in the disclosed invention. However, it is to be understood that the value to which the modifier "about" refers is itself also specifically disclosed.

"Comprise," "comprising," and "comprises" and "comprised of" as used herein are synonymous with "include", "including", "includes" or "contain", "containing", "contains" and are inclusive or open-ended terms that specifies the presence of what follows e.g. component and do not exclude or preclude the presence of additional, non-recited components, features, element, members, steps, known in the art or disclosed therein.

The recitation of numerical ranges by endpoints includes all numbers and fractions subsumed within that range, as well as the recited endpoints.

The expression "% by weight" (weight percent), here and throughout the description unless otherwise defined, refers to the relative weight of the respective component based on the overall weight of the formulation.

As used herein the terms "thermoplastic sheet material" and "thermoplastic sheet" are synonyms.

In a first aspect, the present invention provides a thermoplastic sheet for use in radiotherapy treatment, comprising a thermoplastic composition layer comprising a mixture of polycaprolactone and polyurethane, said layer is provided with an upper surface and a lower surface; and a support layer bonded on at least one surface of the thermoplastic composition layer, said support layer has the thickness from 80 µm to 180 µm and comprises silicone, wherein the thickness of the thermoplastic composition layer is from 1.4 mm to 1.8 mm and whereby the radiation transmission factor of the thermoplastic sheet is at least 80%. The thermoplastic composition layer may comprise polycaprolactone as main component.

In a preferred embodiment, the present invention provides a thermoplastic sheet for the production of a personalized facial mask for immobilizing a person's head for radiotherapy treatment. The personalized mask is produced by directly molding the thermoplastic sheet on the patient's face.

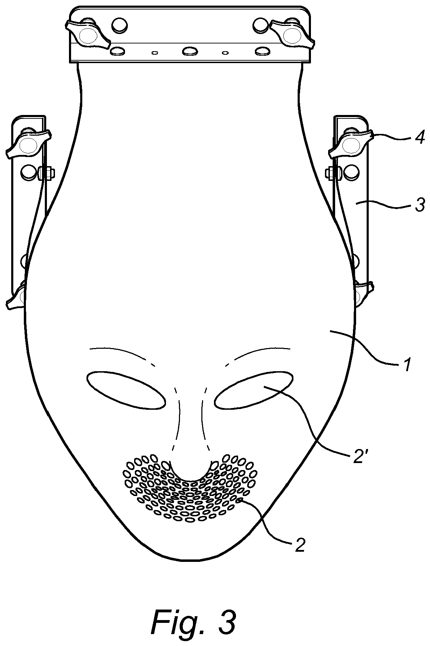

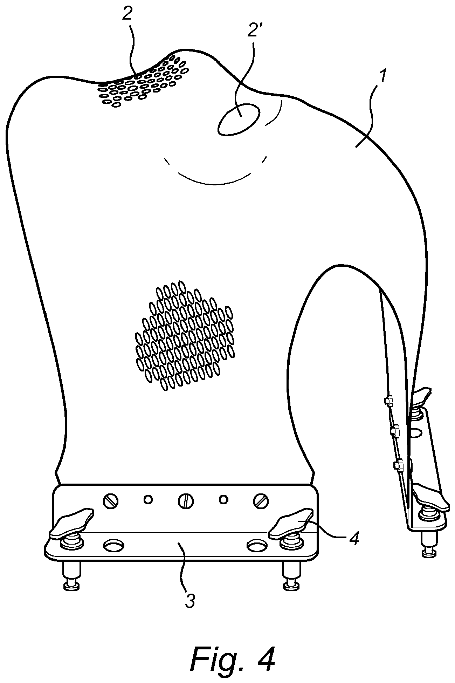

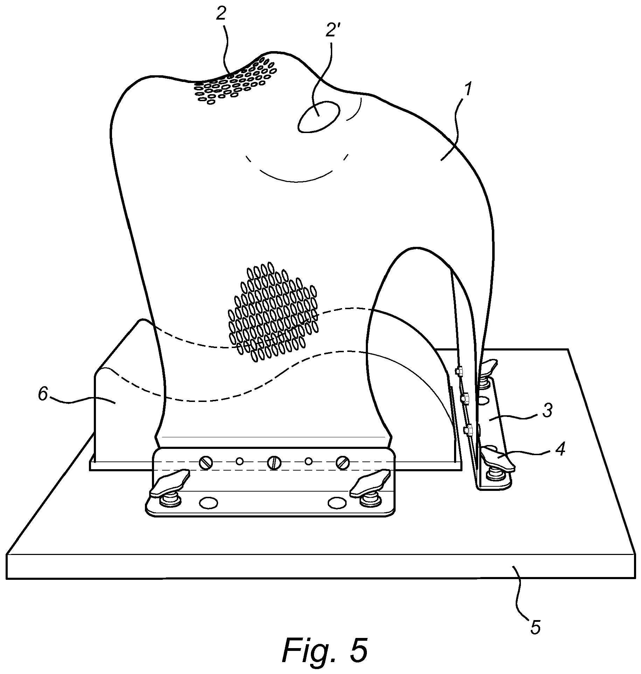

An example of a mask obtained by molding a thermoplastic sheet directly on a person's face is shown in the figures. **Figure 3** shows a top view of the mask or the molded sheet. Said mask **1** is connectable to at least one fixation element **3** for attaching the mask to a fixation surface or a fixation table. The fixation element **3** comprises at least one fastening means **4** such as rivets for attaching the mask to the fixation surface. The mask shown in **Figure 3** is provided with spaced apart groups of perforations **2.** It is to be understood that perforations can be provided in a different pattern, uniformly distributed over the full mask or the mask can be devoid of perforations. **Figure 4** shows a side view of the molded sheet shown in **Figure 3****.** Perforations **2** corresponding to the ears and mouth areas are shown in this example. The sheet may also comprise openings **2'** corresponding to the eyes areas as shown in the figures. **Figure 5** shows an embodiment of the invention wherein the sheet of **Figure 4** is fixed to a fixation surface **5** via the fixation elements **3** and the fastening means **4.** As shown in the same figure, a cushion **6** may be used for immobilizing a patient's head.

### Thermoplastic sheet

By the term "thermoplastic sheet" as used herein, is understood a sheet that is deformable under the application of heat and after cooling down retains the deformed shape and becomes rigid. The overall thickness of the thermoplastic sheet before molding is at most 1.8 mm, most preferably at most 1.6 mm. The thickness of the thermoplastic sheet in mm is preferably 1.4, 1.41, 1.45, 1.5, 1.51, 1.55, 1.6, 1.61, 1.65, 1.7, 1.71, 1.75, 1.8 or a value in the range between any two of the aforementioned values.

In a preferred embodiment, the thickness of the sheet according to the present invention is equal to the sum of the thickness of the thermoplastic composition layer and the thickness of the support layer. The thickness of the thermoplastic composition layer is preferably about 1.6 mm and the thickness of the support layer is of from 100 µm to 150 µm.

The radiation transmission factor of the thermoplastic sheet is at least 80%, preferably at least 85%, more preferably at least 90%, even more preferably at least 95%, most preferably at least 99% or a value in the range between any two of the aforementioned values. By radiation transmission factor, reference is made to the transmittance of the sheet, so to the radiation percentage that traverses the sheet without trajectory deviation or scattering. Said radiation transmission factor is relevant for the sheet dosimetry, i.e. the radiation dose received by the patient which body part is immobilized using the sheet of the invention. The sheet of the present invention is characterized by a high radiation transmission factor thereby ensuring a correct radiation dosimetry and an effective treatment of the patient by radiotherapy. The radiation transmission factor is measured as described in the examples.

In addition, the relatively low thickness of the thermoplastic sheet and/or the presence of the support layer allow a better conformance of the thermoplastic sheet to the anatomical contours of the patient's body part during molding. An excessive pressure applied is not required for achieving a good conformance to the body part anatomical contours using the thermoplastic sheet of the invention. The sheet is at the same time rigid enough to maintain the molded shape and to considerably reduce and even prevent retraction after molding. The rigidity of the sheet of the present invention is comparable and similar to the rigidity of a thermoplastic sheet made of polycaprolactone or a mixture of polycaprolactone and polyurethane and having a thickness of 2.3 mm.

In a preferred embodiment, the shore A of the thermoplastic sheet before heating and/or molding is at least 90, preferably at least 91, more preferably at least 92, most preferably at least 94. The shore A is at most 99, preferably at most 98, more preferably at most 97, most preferably 96. Preferably, the shore A is about 94. The shore A was measured according to the standard Din 53505.

The thermoplastic sheet according to the invention is by preference moldable at temperatures of between 40°C and 90°C, more by preference between 50°C and 70°C. This temperature is depending on the polycaprolactone content of the sheet. The sheet will remain in a plastic condition until it is cooled to about or below 35°C. Typically the sheet is brought into the moldable state by heating in a water bath set around 5°C above the melting temperature, most preferable at 65°C.

The thermoplastic sheet may be provided with perforations or devoid of perforations. The perforations can be distributed over the full surface of the sheet. Preferably the perforations are arranged in spaced apart groups. More preferably, the groups of perforations are positioned such that they at least correspond to the eyes and/or the mouth and/or the ears of a person. This provides aeration and breathing to the patient with minimal effect on the dosimetry and the radiation transmission factor. In addition, the patient is provided with continuous connection with the surrounding environment thereby increasing his comfort. An example of spaced apart groups of perforations are shown in the appended figures and referred to by number **2.**

The perforations cover about 95%, 90%, 80%, 70%, 60%, 50%, 40%, 30%, 20%, 10 or 5% of the sheet total area or a value in the range between any two of the aforementioned values. The perforations may have a diameter of 0.4 mm, 0.5 mm, 0.6 mm, 0.7 mm, 0.8 mm, 0.9 mm, 1 mm, 1.1 mm, 1.2 mm, 1.3 mm, 1.4 mm, 1.5 mm, 1.6 mm, 1.7 mm, 1.8 mm, 1.9 mm, 2.0 mm, 2.1 mm, 2.2 mm, 2.3 mm, 2.4 mm, 2.5 mm, 2.6 mm, 2.7 mm, 2.8 mm, 2.9 mm, 3 mm or a value in the range between any two of the aforementioned values.

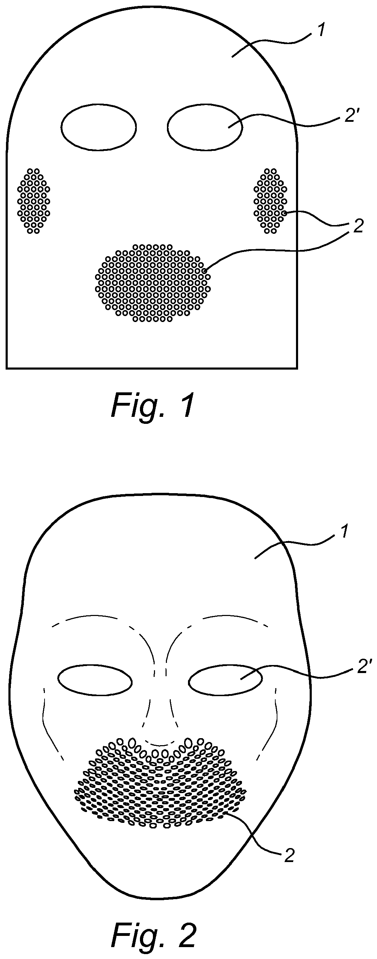

The thermoplastic sheet, according to the present invention, can directly be molded on a person body part, such as the face and/or the neck and/or the shoulders, whereby the support layer will be the skin-facing layer. The resulting mask is suitable to be used for the immobilisation of the person's body part for radiotherapy treatment. Said mask provides a comfortable wearing to the patient. The sheet exhibits excellent deformability properties, conforming to the shape of the face without the need to apply excessive pressure. The silicone layer provides a comfortable wearing against the skin. Prior molding, the mask is provided as a pre-cut thermoplastic sheet. Said pre-cut sheet is provided in standard or variable dimensions. An example of the sheet **1** according to an embodiment of the invention is shown in **Figure 1****.** During molding a patient's body part, the heat impact on the skin is considerably reduced thereby greatly increasing the patient's comfort.

In a preferred embodiment, the mask of the present invention is reusable from 2 to 30 times, preferably from 5 to 25 times, more preferably from 10 to 20 times. Preferably, during heating of the thermoplastic sheet, a signal is provided to the user or the practitioner when the temperature of the sheet is appropriate for molding. Said signal is preferably a visual signal such as a change of the color of the sheet.

### Thermoplastic composition layer

By the term "thermoplastic composition layer" as used herein, is understood a layer comprising a thermoplastic composition having two surfaces: an upper surface and a lower surface. The thermoplastic composition may be chosen from the group of polycaprolactone and polyurethane or any combination thereof.

The polyurethane may be present in an amount of 0%, 10%, 20%, 30%, 40% or 50% (% by weight), preferably 20% to 40% or a value in the range between any two of the aforementioned values, most preferably 30%. The polycaprolactone may be present in an amount of 60%, 70%, 80%, or 90% (% by weight) or a value in the range between any two of the aforementioned values, preferably 60% to 80%, most preferably 70%. Typically, there will be more polycaprolactone than polyurethane which polycaprolactone lowers the temperature at which the sheet deforms. The ratio of polycaprolactone:polyurethane (weight: weight) may be 5:1, 4:1, 3:2, 3:1, 2.3:1, 2:1 preferably 2.3:1.

The molecular weight of the polyurethane may be equal to or less than 10000, 20000, 30000, 40000, 50000, 60000, 70000, 80000, 90000, 100000, 120000, 140000, 150000 or a value in the range between any two of the aforementioned values, preferably between 10000 and 100000. Polyester polyurethane is the preferred polyurethane.

The molecular weight of the polycaprolactone may be 10000, 20000, 30000, 40000, 50000, 60000, 70000, 80000, 100000, 200000, 300000, 400000, 500000 or a value in the range between any two of the aforementioned values, preferably between 10000 and 60000, more preferably between 37000 and 500000.

Caprolactone polyester polyurethane is particularly suitable, which polyurethane may be obtained by reacting isocyanate and polycaprolactone-based polyester. Such a caprolactone polyester polyurethane is commercially available as granulate. The melting point of said polycaprolactone polyester polyurethane lies between 140°C and 180°C. By adding the polycaprolactone, also preferably in granulate form, a thermoplastic composition is obtained that is distortable and moldable at a temperature of about 69°C and remains distortable by cooling down to about or below 35°C.

At this temperature, the thermoplastic composition layer may be stretched at least up to four times the original length thereof. In the hardened condition, the thermoplastic sheet is rigid and has a memory effect that, after heating, said sheet returns to its original shape, so to its shape before molding. It is non-elastic in the hardened condition. The thermoplastic composition layer of the present invention is used to be directly molded on a human body part thereby obtaining anatomical shaped medical articles that conform the anatomical contours of said body part. The sheet of the invention has increased stiffness or rigidity after cooling down.

The thermoplastic composition layer may have a thickness of 0.8 mm, 1.0 mm, 1.5 mm, 1.6 mm, 1.7 mm, 1.8 mm or a value in the range between any two of the aforementioned values. This relatively low thickness of the thermoplastic composition layer contributes to the outstanding good conformance of the thermoplastic sheet to the anatomical contours of the patient's body part during molding. An excessive pressure applied is not required for achieving a good conformance to the body part anatomical contours using the thermoplastic sheet of the invention. A coloring agent may be added to the thermoplastic composition layer.

The thermoplastic composition layer is preferably perforated to increase breathability. The perforations may have a diameter of 0.4 mm, 0.5 mm, 0.6 mm, 0.7 mm, 0.8 mm, 0.9 mm, 1 mm, 1.1 mm, 1.2 mm, 1.3 mm, 1.4 mm, 1.5 mm, 1.6 mm, 1.7 mm, 1.8 mm, 1.9 mm, 2.0 mm 2.1 mm, 2.2 mm, 2.3 mm, 2.4 mm, 2.5 mm, 2.6 mm, 2.7 mm, 2.8 mm, 2.9 mm, 3 mm or a value in the range between any two of the aforementioned values. Said thermoplastic composition layer may also be devoid of perforations.

In a preferred embodiment, the tensile strength of the thermoplastic composition layer having perforations is of from 11 to 16 MPa, preferably from 12 to 15 MPa or any value comprised between two of the aforementioned values. The tensile strength of the thermoplastic composition layer devoid of perforations is of from 17 to 24 MPa, preferably from 18 to 23 MPa or any value comprised between two of the aforementioned values. The tensile strength is determined according to EN ISO 527-2.

In a preferred embodiment, the strain at break of the thermoplastic composition layer having perforations is of from 1350 to 1600%, preferably from 1370 to 1550% or any value comprised between two of the aforementioned values. The tensile strength of the thermoplastic composition layer devoid of perforations is of from 1470 to 1900%, preferably from 1480 to 1870% or any value comprised between two of the aforementioned values. The tensile strength is determined according to EN ISO 527-3.

The physical properties of the perforated and non-perforated thermoplastic composition layer according to an embodiment of the invention were measured. The results are shown in table 1 below. Said results were obtained from the average of 3 separate measurements. By thermoplastic composition layer, we refer to the thermoplastic sheet which is not covered by the support layer.

**Table 1: physical properties of the perforated and non-perforated thermoplastic composition layer**

| | Perforated sheet | Non-perforated sheets | Measurement method |
|---|---|---|---|
| Thickness in mm | 1.7 | 1.7 | |
| Density in g/cm³ | 1.14 | 1.14 | ISO 2781 |
| Elastic modulus in MPa | 23.3 | 23.3 | LST EN ISO 527-1 |
| Tensile strength in MPa | 13.2 | 19.9 | LST EN ISO 527-2 |
| Strain at break in % | 1480 | 1728 | LST EN ISO 527-3 |
| Flexural modulus in MPa | 275 | 460 | LST EN ISO 178 |
| Maximum elongation in %* | 1240 | 739 | LST EN ISO 527 |

| | | | |
|---|---|---|---|
| * maximum elongation refers to the length or the sheet when it is elongated compared to its original length before elongation | | | |

A non-limiting example of a thermoplastic composition that can be used in the current invention is Turbocast® (T Tape Company BV).

### Support layer

By the term "support layer" is meant a layer that comprises a composition different from the thermoplastic composition layer. In a preferred embodiment, the thermoplastic composition layer comprises a support layer only on one of its surfaces. The thermoplastic composition layer may also comprise support layers on each of its surfaces. The support layer comprises silicone and preferably consists of silicone. The support layer is preferably directly bound to at least one surface of the thermoplastic composition layer. By directly bound it is meant that the support layer is in direct contact with the surface of the thermoplastic composition layer.

The advantage of using silicone as support layer or as part of said support layer is that said silicone will act as a friction restraining material and will provide comfort to the wearer when the silicone side is the skin facing side of the sheet. Silicone reduces the friction between the sheet and the skin. Another advantage of silicone is its heat resistance capacity; this will buffer the heat of the thermoplastic sheet when it is directly molded on the skin. Another advantage of silicone is that it is vapor-permeable. Furthermore, a major advantage of using silicone is that it creates a micro-climate where it is applied. Such micro-climate enhances penetration into the skin of the patient of compounds and/or active molecules present in the silicone. Furthermore, said micro-climate will allow for an accelerated healing of burns and or dermatologic scars and it will lessen permanent scars on the skin. By treatment, we refer to medical treatments or any other treatments known to the person skilled in the art and requiring the immobilization of at least a part of the patient's body, in particular radiotherapy.

The use of support layer comprising silicone and preferably consisting of silicone contributes to the outstanding good molding properties of the thermoplastic sheet of the present invention. Indeed, when the sheet is heated and applied on the person's body part, the support layer adheres quickly to the skin thanks to the presence of the silicone. The silicone is quickly attracted by the patient's skin and quickly conforms to the anatomical contours of the body part to be immobilized. The presence of the silicone and the thickness of the thermoplastic composition layer provide extreme good molding properties of the sheet and a short molding time. The molded sheet perfectly conforms the anatomical contours of the body part thereby considerably improving the immobilization process and the repetitive treatment efficiency. The short molding time improves patient's comfort and practitioner's work conditions.

In a preferred embodiment, the support layer freely and perfectly follows the deformations of the thermoplastic composition layer, during and after molding of the thermoplastic sheet but also during the hardening of the thermoplastic sheet after molding. When the thermoplastic composition is rigid, the support layer is fully adapted to the form of the thermoplastic sheet.

In a preferred embodiment, the support layer is hydrophobic and/or is provided with low thermal conductivity. The support layer comprising silicone is therefore heat resistant and buffers the heat of the thermoplastic sheet when it is directly molded on the skin. This avoids applying warm sheet on the patient's skin and considerably limits skin irritation during the molding of the sheet.

In a preferred embodiment, the support layer is at least partially polymerized. Preferably, the silicone of the support layer is partially polymerized.

The thickness of the support layer **6** is from 80 µm to 180 µm. The silicone further enhances the memory effect of the thermoplastic sheet. Silicone applied in relatively thin layer to the thermoplastic sheet provides more elasticity to the sheet thereby enhancing its memory effect. In addition, the thickness of the support layer as provided by the invention does not affect the radiation transmission factor and/or the dosimetry of the thermoplastic sheet.

The silicone is preferably non-adhesive. The silicone can also be adhesive on at least one of its sides. The adhesive side can then be used to bond the silicone to the thermoplastic composition layer. The silicone can also be partially adhesive thereby comprising adhesive and non-adhesive areas. Preferably, the silicone is translucent.

In a preferred embodiment, the silicone is provided with high tear strength. Preferably, the gravity of said silicone, determined according to ASTM D792, is of from 0.5 to 2 gr/cm³, more preferably from 0,8 to 1.5 gr/cm³, most preferably about 1 gr/cm³.

In a preferred embodiment, the shore A of the silicone, determined according to ASTM D2240, is of from 40 to 100, preferably from 45 to 85, more preferably from 50 to 70, most preferably about 60.

In a preferred embodiment, the tensile strength of the silicone, determined according to ASTM D412, is of from 5 to 12 Mpa, preferably from 6 to 10 Mpa, more preferably from 7 to 9 Mpa, most preferably about 8 Mpa.

In a preferred embodiment, the tear strength of the silicone, determined according to ASTM D624 - method A, is of from 10 to 25 KN/m, preferably from 12 to 20 KN/m, more preferably from 14 to 17 KN/m, most preferably about 15 KN/m.

In a preferred embodiment, the elongation of the silicone, determined according to ASTM D412, is of from 400 to 900%, preferably from 500 to 800%, more preferably from 600 to 700%.

In a preferred embodiment, the tack free time, determined according to ASTM C679 / NT-TM005, is of from 2 to 20 min, preferably from 5 to 18 min, more preferably from 8 to 16 min, most preferably from 10 to 12 min.

In a preferred embodiment, the temperature of fragility of the silicone is of from 100 to 400°C, preferably from 150 to 350°C, more preferably from 180 to 250°C, most preferably from 200 to 220°C.

In a preferred embodiment, the thermoplastic composition layer and/or the support layer comprises a compound selected from the group comprising silver ions, silver bromide, silver carbonate, silver chloride, silver nitrate, silver sulfadiazine, a biocide, an anti-bacterial or any combination thereof. In a preferred embodiment, the thermoplastic sheet comprises about 3% of said compound. In a preferred embodiment, the support layer consists of silicone and any of the aforementioned compounds. The support layer may also contain other metals such as gold (in form of glitters or filaments), platinum, zirconium, copper or any combination thereof.

In an embodiment of the invention, the support layer is itself coated with a polymer. By preference, the polymer is chosen from the group comprising but not limited to elastomers such as ethylene-vinyl acetate, polyester, breathable polyurethane; and synthetic silicone. The support layer may be coated on one side or on both sides. The advantage of using polymer coating is that it will freely follow the expansion and contraction of the thermoplastic sheet.

In a preferred embodiment of the invention, the support layer optionally comprises a backing layer which is removed before application to the subject. The backing layer reversibly adheres to the support layer intended for contact with the skin of the subject. The backing layer may be made of any suitable material such as, for example, polyethylene, polythene or other polymeric substance. According to an aspect of the invention, the backing layer is smooth, or is regularly patterned with pits or grooves. In another preferred embodiment of the invention, the coating optionally comprises a backing layer. Said backing layer of the coating is similar to the one described for support layer.

In another embodiment of the invention, the support layer can be impregnated. By preference, the support layer is impregnated with a polymer. More by preference, the polymer is chosen from the group comprising but not limited to elastomers such as ethylene-vinyl acetate and polyester.

In a preferred embodiment, the support layer is bonded on at least one surface of the thermoplastic composition layer. In a preferred embodiment, said support layer comprises silicone. Preferably, said support layer comprises a silicone layer which is directly bound to at least one surface of the thermoplastic composition layer. The support layer is in direct contact with the surface of the thermoplastic composition layer.

In a preferred embodiment, the support layer is sprayed on the thermoplastic composition layer, thereby coating at least one surface of said thermoplastic composition layer. Said spraying can be performed at room temperature. Spraying can be performed using any method known to the person skilled in the art. Preferably, the support layer is sprayed on said thermoplastic composition layer using a spray gun. Using spray method, a thin support layer can be easily created on the thermoplastic composition layer. This is not offered by other bonding methods wherein handling of thin support layers is delicate and leads to irregularities in the final produced material.

In a preferred embodiment, silicone is sprayed on the thermoplastic composition layer and is allowed to cool and dry thereby forming a first layer of silicone covering at least one surface of the thermoplastic composition layer. A second layer of silicone can be created on the silicone layer which is already obtained. Said second layer is also created by spraying silicone using a spray gun. Said second layer with said first layer determine the thickness of the support layer applied on the thermoplastic composition layer. This provides a control over the total thickness of the thermoplastic sheet.

In a preferred embodiment, the support layer is deposited on the thermoplastic composition layer. After deposit, the support layer is scraped using a scraping means thereby coating at least one surface of said thermoplastic composition layer with an equally distributed support layer. The support layer might be deposited by spraying it on the thermoplastic composition layer. Here also, a thin support layer can be easily created on the thermoplastic composition layer.

In a preferred embodiment, the support layer is applied on the thermoplastic composition layer by airless spraying, using a pistol or using a roll.

In a preferred embodiment, the thermoplastic composition layer is treated with an adhesion primer prior to the application and/or the creation of the support layer. Said primers enhance the adhesion of the support layer to the thermoplastic composition layer. Said primers can be of chemical and/or mechanical nature or any other type known to the person skilled in the art.

In a preferred embodiment of the invention, 70%, preferably 80%, more preferably 90%, most preferably 100% of the surface area of at least one side of the thermoplastic composition layer is coated by the support layer. Both sides of the thermoplastic composition layer might be coated by the support layer.

In the method of the present invention, the support layer comprising silicone might be applied to the thermoplastic composition layer using different techniques. Said support layer might be applied by screen printing or by roller coating.

The present invention further provides a method for the production of a thermoplastic sheet as described above, comprising the step of bonding the support layer to at least one surface of the thermoplastic composition layer using ultraviolet (UV) curing. In a preferred embodiment, the support layer comprises silicone. Preferably, the support layer comprises a silicone layer which is directly bound to at least one surface of the thermoplastic composition layer. The support layer is in direct contact with the surface of the thermoplastic composition layer.

A silicone layer is placed on at least one surface of the thermoplastic composition layer. The laminated structure is placed under UV lights to be cured. Preferably, the curing time is of from 10 to 120 seconds, more preferably from 20 to 100 seconds, most preferably from 30 sceonds to 80 seconds, most preferably from 40 to 60 seconds. Amongst the advantages of UV curing are the facility of the application and treatment and the short operation time which increases production speed. In addition, using UV curing method, there is no need for heating the laminate thereby preserving the thermoplastic composition layer from any deformation. Said thermoplastic composition layer being moldable at low temperatures as mentioned above.

In a second aspect, the present invention provides a method for the production of a thermoplastic sheet. The sheet is as described above and the method comprises the steps of:
- producing a thermoplastic composition layer comprising a mixture of polycaprolacone and polyurethane,
- treating at least one surface of the thermoplastic composition layer thereby increasing its surface adherence,
- applying at least one layer of coating on the treated thermoplastic composition surface, and
- creating spaced apart groups of perforations.

In a preferred embodiment, the thermoplastic composition layer and/or the support layer are as described above.

In a preferred embodiment, at least one surface of the thermoplastic composition layer is treated with an adhesion primer prior to the application and/or the creation of the support layer. Said primers enhance the adhesion of the support layer to the thermoplastic composition layer. Said primers can be of chemical and/or mechanical nature or any other type known to the person skilled in the art. Other surface treatments may also be used such as isopropanol or plasma treatment.

In a preferred embodiment of the invention, 70%, preferably 80%, more preferably 90%, most preferably 100% of the surface area of at least one side of the thermoplastic composition layer is coated by the support layer. Both sides of the thermoplastic composition layer might also be coated by the support layer.

Further provided is the use of a thermoplastic sheet as described in any embodiment of the invention for molding a mask directly on a person body part, whereby the support layer is the skin-facing layer.

The thermoplastic sheet according to any embodiment of the invention is suitable for use in the oncology field as well as for the treatment of burns and/or dermatological scars. Additionally, the support layer may contain anti-bacterial products as described above and/or be treated with cosmetic products, suitable to be used in cosmetic therapy.

In another embodiment of the invention, a thermoplastic sheet according to the invention is suitable to be used for treatment of dermatological scars after breast operations. A thermoplastic sheet can be included in a bra whereby the silicone layer is facing the skin of the patient. The inclusion of a thermoplastic sheet according to the invention in a bra will fasten the healing process of the skin after such operation and will lessen the amount and grade of the scars.

The thermoplastic sheet may be pre-formed prior to molding. In an embodiment of the invention, the thermoplastic sheet may be sealed prior to use. This sealing is preferably sterile, vacuum and water-repellent. Such embodiment is suitable to use for treatment of burns and dermatological scars. Such embodiment of the thermoplastic sheet is by preference heated while hermetically sealed and opened in a sterile environment just before application to the body part to be treated. The sealing is by preference watertight.

The invention further provides a kit for radiotherapy comprising at least one thermoplastic sheet as described above and at least one leaflet comprising use instructions. The kit might further comprise fixation elements to fix and attach the sheet to a fixation surface or a fixation table.

### EXAMPLES

Comparative Example **1:** Thermoplastic sheet: total thickness 1.6 mm A thermoplastic composition layer was made with the following properties:
- material: 30% polyester polyurethane, 70% polycaprolactone
- melting point: 200°C
- perforations: 0.7mm diameter

This thermoplastic composition layer was sprayed on one surface with silicone having the following properties:
- material: synthetic silicone
- coating weight: 12 g/m²
- application: sprayed on one side of the thermoplastic composition layer.

The radiation transmission factor of the final obtained sheet was determined according to the method described below.

Comparative sheets were made. Said sheet were devoid of silicone and had a total thickness of 2 mm and 2.2 mm. The radiation transmission factor of said sheets was also determined according to the same method.

The radiation transmission factor was measured using an Elektra accelerator (Elektra Precise with MLCi- collimator, 6 and 10 MV Photon radiation). The used phantom was a Perspex cylindrical phantom with a diameter of 10 cm. The used ionization chamber was a Farmer NE-2571 with serial number 3593. The used electrometer was PWT Unidos Webline with high voltage of -250V. The measuring depth was 5 cm. The number of monitor units per measurement was 100 MU.

The measurements were executed with 6 MV or 10 MV photon beam with 4 different field sizes: 3X3, 5X5, 8X8 and 10X10 under 2 gantry angles: 0° and 45°. The radiation transmission factor of each sheet was determined for each field size, under each angle and each photon beam. The obtained results corresponding to the 4 different field sizes were averaged and are reproduced in Table 1 below:

**Table 1: average radiation transmission factor**

| | | Sheet 1.6 mm perforated | Sheet 1.6 mm non-perforated | Sheet 2 mm perforated | Sheet 2.2 mm perforated |
|---|---|---|---|---|---|
| 6MV | 0° | 99.4% | 99.4% | 99.2% | 99.1% |
| | 45° | 99.1% | 98.8% | 98.1% | 98.1% |
| 10MV | 0° | 99.6% | 99.5% | 99.4% | 99.3% |
| | 45° | 99.5% | 99.2% | 98.5% | 98.5% |

The radiation transmission factor of the sheet of the present invention was always higher compared to the radiation transmission factor of the comparative sheets. The difference in transmission factors percentages is very significant in the radiotherapy field.

The Buildup skin dose (dosimetry) was measured using an Elektra accelerator (Elektra Precise with MLCi- collimator, 6 and 10 MV Photon radiation). The used phantom was a RW3 solid water phantom. The used ionization chamber was a Farmer Roos chamber. The used electrometer was PTW Unidos Webline with a high voltage of -250V. The measuring depth was variable. The number of monitor units per measurement was 100 MU. The measurements were executed with 6MV or 10 MV Photon beam with 1 field size, i.e. 10x10 under 1 gantry angle, i.e. 0°.

The Percentage Depth Dose (PDD) graphs with and without sheet were made (not shown). By making PDD graphs on the phantom block, the influence of the sheet on the dose buildup at the skin was determined. The obtained values of skin dose buildup are shown in table 2.

**Table 2: Buildup skin dose values, expressed in mm equivalent of water**

| | Sheet 1.6 mm perforated | Sheet 1.6 mm non-perforated | Sheet 2 mm perforated | Sheet 2.2 mm perforated |
|---|---|---|---|---|
| 6MV | 1.5 | 1.46 | 2.3 | 2.37 |
| 10MV | 1.91 | 1.82 | 2.16 | 2.22 |

The buildup skin values of the sheet of the present invention are lower compared to those of the comparative sheets. This shows that the percentage depth dose that reaching the inner tissues of the organ receiving the radiotherapy is higher when the sheet of the present invention is used.

## Claims

1. A radiotherapy thermoplastic sheet, comprising a thermoplastic composition layer comprising a mixture of polycaprolactone and polyurethane, said layer is provided with an upper surface and a lower surface; and a support layer provided on at least one surface of the thermoplastic composition layer, said support layer comprises silicone which is at least partially polymerized, **characterized in that,**
the thickness of the thermoplastic composition layer is at most 1.8 mm,
the thickness of the support layer is of from 80 µm to 180 µm, and
the radiation transmission factor of the sheet, as measured by an Elektra accelerator according to the method described in example 1, is at least 80%.

2. The sheet according to claim 1, wherein the support layer is hydrophobic.

3. The sheet according to any of claims 1-2, wherein the radiation transmission factor of the sheet is at least 90%.

4. The sheet according to any of claims 1-3, comprising spaced apart groups of perforations.

5. The sheet according to claim 4, wherein the groups of perforations are positioned such that they at least correspond to the eyes and/or the mouth and/or the ears of a person.

6. The sheet according to any of claims 1-5, wherein the support layer and/or the thermoplastic sheet comprises a compound selected from the group comprising silver ions, silver bromide, silver carbonate, silver chloride, silver nitrate, silver sulfadiazine, a biocide, an anti-bacterial or any combination thereof.

7. The sheet according to any of the previous claims 1-6, whereby the sheet is moldable at a temperature comprised between 40°C and 90°C, preferably between 50°C and 70°C and remains in plastic condition until cooled below 35°C.

8. The sheet according to any of claims 1-7, whereby the sheet is rigid at hardened condition.

9. A method for the production of a radiotherapy thermoplastic sheet as described in any of claims 1-8 comprising the steps of:
- producing a thermoplastic composition layer comprising a mixture of polycaprolacone and polyurethane,
- treating at least one surface of the thermoplastic composition layer thereby increasing its surface adherence,
- applying a support layer on the treated thermoplastic composition surface wherein said support layer comprises silicone which is at least partially polymerized, and
- creating spaced apart groups of perforations,
**characterized in that,**
the thickness of the thermoplastic composition layer is of from 1.4 to 1.8 mm, and the thickness of the support layer is of from 80 µm to 180 µm.

10. Use of a thermoplastic sheet according to any of claims 1-8, for molding a mask directly on a person body part, whereby the support layer is the skin-facing layer.

11. A kit comprising at least one thermoplastic sheet as described in any of claims 1-8 and at least one leaflet comprising use instructions.

## Patentansprüche

1. Strahlentherapie-Thermoplastbahn, eine Schicht aus einer Thermoplastzusammensetzung, die eine Mischung aus Polycaprolacton und Polyurethan umfasst, wobei die Schicht mit einer oberen Oberfläche und einer unteren Oberfläche versehen ist, und eine Trägerschicht umfassend, die auf mindestens einer Oberfläche der Schicht aus einer Thermoplastzusammensetzung bereitgestellt ist, wobei die Trägerschicht Silikon umfasst, das zumindest teilweise polymerisiert ist, **da**
**durch gekennzeichnet, dass**
die Dicke der Schicht aus einer Thermoplastzusammensetzung höchstens 1,8 mm beträgt,
die Dicke der Trägerschicht zwischen 80 µm und 180 µm beträgt und
der Strahlungsübertragungsfaktor der Bahn, gemessen von einem Elektra-Beschleuniger gemäß dem in Beispiel 1 beschriebenen Verfahren, höchstens 80 % beträgt.

2. Bahn nach Anspruch 1, wobei die Trägerschicht hydrophob ist.

3. Bahn nach einem der Ansprüche 1-2, wobei der Strahlungsübertragungsfaktor der Bahn mindestens 90% beträgt.

4. Bahn nach einem der Ansprüche 1-3, beabstandete Gruppen von Perforationen umfassend.

5. Bahn nach Anspruch 4, wobei die Gruppen von Perforationen derart positioniert sind, dass sie mindestens den Augen und/oder dem Mund und/oder den Ohren einer Person entsprechen.

6. Bahn nach einem der Ansprüche 1-5, wobei die Trägerschicht und/oder die Thermoplastbahn eine Verbindung umfasst/umfassen, die aus der Gruppe ausgewählt ist, die Silberionen, Silberbromid, Silbercarbonat, Silberchlorid, Silbernitrat, Silbersulfadiazin, ein Biozid, ein antibakterielles Mittel oder eine Kombination daraus umfasst.

7. Bahn nach einem der Ansprüche 1-6, wobei die Bahn bei einer Temperatur zwischen 40 °C und 90 °C formbar ist, vorzugsweise zwischen 50 °C und 70 °C, und in plastischem Zustand bleibt, bis sie auf unter 35 °C gekühlt wird.

8. Bahn nach einem der Ansprüche 1-7, wobei die Bahn in einem gehärteten Zustand starr ist.

9. Verfahren zur Herstellung einer Strahlentherapie-Thermoplastbahn nach einem der Ansprüche 1-8, folgende Schritte umfassend:
- Herstellen einer Schicht aus einer Thermoplastzusammensetzung, die eine Mischung aus Polycaprolacton und Polyurethan umfasst,
- Behandeln mindestens einer Oberfläche der Schicht aus einer Thermoplastzusammensetzung, wodurch deren Oberflächenhaftvermögen erhöht wird,
- Aufbringen einer Trägerschicht auf die behandelte Oberfläche der Thermoplastzusammensetzung, wobei die Trägerschicht Silikon umfasst, das zumindest teilweise polymerisiert ist, und
- Erzeugen beabstandeter Gruppen von Perforationen,
**dadurch gekennzeichnet, dass**
die Dicke der Schicht aus einer Thermoplastzusammensetzung von 1,4 bis 1,8 mm beträgt und die Dicke der Trägerschicht zwischen 80 µm und 180 µm beträgt.

10. Verwendung einer Thermoplastbahn nach einem der Ansprüche 1-8 zum Formen einer Maske direkt auf einem Körperteil einer Person, wobei die Trägerschicht die der Haut zugewandte Schicht ist.

11. Set, umfassend mindestens eine Thermoplastbahn nach einem der Ansprüche 1-8 und mindestens einen Handzettel, der Anwendungsanweisungen umfasst.

## Revendications

1. Feuille thermoplastique de radiothérapie, comprenant une couche de composition thermoplastique comprenant un mélange de polycaprolactone et de polyuréthane, ladite couche est pourvue d'une surface supérieure et d'une surface inférieure ; et une couche de support prévue sur au moins une surface de la couche de composition thermoplastique, ladite couche de support comprend du silicone qui est au moins partiellement polymérisé, **caractérisée en ce que,**
l'épaisseur de la couche de composition thermoplastique est d'au plus 1,8 mm,
l'épaisseur de la couche de support est de 80 µm à 180 µm, et
le facteur de transmission de rayonnement de la feuille, tel que mesuré par un accélérateur Elektra selon le procédé décrit dans l'exemple 1, est d'au moins 80 %.

2. Feuille selon la revendication 1, dans laquelle la couche de support est hydrophobe.

3. Feuille selon l'une quelconque des revendications 1-2, dans laquelle le facteur de transmission de rayonnement de la feuille est d'au moins 90 %.

4. Feuille selon l'une quelconque des revendications 1-3, comportant des groupes de perforations espacés.

5. Feuille selon la revendication 4, dans laquelle les groupes de perforations sont positionnés de telle sorte qu'ils correspondent au moins aux yeux et/ou à la bouche et/ou aux oreilles d'une personne.

6. Feuille selon l'une quelconque des revendications 1-5, dans laquelle la couche de support et/ou la feuille thermoplastique comprend un composé choisi dans le groupe comprenant des ions argent, du bromure d'argent, du carbonate d'argent, du chlorure d'argent, du nitrate d'argent, de la sulfadiazine d'argent, un biocide, un agent antibactérien, ou une combinaison quelconque de ceux-ci.

7. Feuille selon l'une quelconque des revendications précédentes 1-6, dans laquelle la feuille peut être moulée à une température comprise entre 40 °C et 90 °C, de préférence entre 50 °C et 70 °C, et reste dans un état plastique jusqu'à un refroidissement inférieur à 35 °C.

8. Feuille selon l'une quelconque des revendications 1 à 7, dans laquelle la feuille est rigide à l'état durci.

9. Procédé pour la production d'une feuille thermoplastique de radiothérapie telle que décrite dans l'une quelconque des revendications 1-8, comprenant les étapes consistant à :
- réaliser une couche de composition thermoplastique comprenant un mélange de polycaprolacone et de polyuréthane,
- traiter au moins une surface de la couche de composition thermoplastique, augmentant ainsi son adhérence superficielle,
- appliquer une couche de support sur la surface de la composition thermoplastique traitée, dans lequel ladite couche de support comprend du silicone qui est au moins partiellement polymérisé, et
- créer des groupes de perforations espacés,
**caractérisé en ce que,**
l'épaisseur de la couche de composition thermoplastique est de 1,4 à 1,8 mm, et l'épaisseur de la couche de support est de 80 µm à 180 µm.

10. Utilisation d'une feuille thermoplastique selon l'une quelconque des revendications 1-8, pour le moulage d'un masque directement sur une partie du corps d'une personne, de sorte que la couche de support est la couche face à la peau.

11. Kit comprenant au moins une feuille thermoplastique telle que décrite dans l'une quelconque des revendications 1-8 et au moins un feuillet comprenant des instructions d'utilisation.
